# EUROPÄISCHE PATENTSCHRIFT

(11) **EP 1 157 014 B1**
(45) Veröffentlichungstag und Bekanntmachung des Hinweises auf die Patenterteilung: **24.08.2005**
(21) Anmeldenummer: 00907504.5
(22) Anmeldetag: 05.02.2000
(51) Int. Cl.: C07D 277/60, C07D 277/84, C07D 417/04, A61K 31/428, A61P 3/04, A61P 3/10

(54) **POLYCYCLISCHE 2-AMINO-DIHYDROTHIAZOLSYSTEME, VERFAHREN ZU IHRER HERSTELLUNG UND IHRE VERWENDUNG ALS ARZNEIMITTEL**
POLYCYCLIC 2-AMINO-DIHYDROTHIAZOLE SYSTEMS, METHOD FOR THE PRODUCTION THEREOF AND THEIR UTILIZATION AS MEDICAMENT
SYSTEMES 2-AMINO-DIHYDROTHIAZOLE POLYCYCLIQUES, LEUR PROCEDE DE PRODUCTION ET LEUR UTILISATION COMME MEDICAMENT

(30) Priorität: 26.02.1999 DE 19908539
(43) Veröffentlichungstag der Anmeldung: 28.11.2001
(73) Patentinhaber: Aventis Pharma Deutschland GmbH, 65929 Frankfurt am Main (DE)
(72) Erfinder: JÄHNE, Gerhard, D-65929 Frankfurt (DE); GEISEN, Karl, D-60318 Frankfurt (DE); LANG, Hans, Jochen, D-65719 Hofheim (DE); BICKEL, Martin, D-61348 Bad Homburg (DE)
(86) Internationale Anmeldenummer: PCT/EP2000/000923
(87) Internationale Veröffentlichungsnummer: WO 2000/051994

(56) Entgegenhaltungen:
- CH-A- 507 290
- DE-A- 2 601 791
- DE-A- 2 640 358
- DE-A- 2 845 857
- US-A- 3 507 868

## Beschreibung

Die Erfindung betrifft polycyclische 2-Amino-Dihydrothiazolsysteme sowie deren physiologisch verträgliche Salze und physiologisch funktionelle Derivate.

Es sind bereits Thiazolidinderivate mit anorektischer Wirkung im Stand der Technik beschrieben (Österreichisches Patent Nr. 365181).

Der Erfindung lag die Aufgabe zugrunde, weitere Verbindungen zur Verfügung zu stellen, die eine therapeutisch verwertbare anorektische Wirkung entfalten.

Die Erfindung betrifft daher Verbindungen der Formel I, 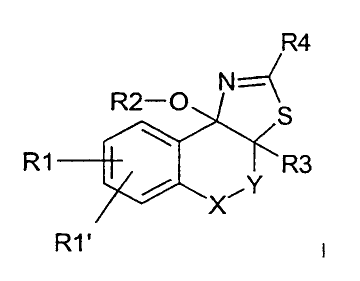 worin bedeuten
- Y: eine direkte Bindung, -CH₂-, -CH₂-CH₂-;
- X: CH₂, CH(CH₃), CH(C₂H₅), CH(C₃H₇), CH(C₆H₅);
- R1, R1': unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; NH₂, NH-(C₁-C₆)-Alkyl, N((C₁-C₆)-Alkyl)₂, NH(C₁-C₇)-Acyl, Phenyl, Biphenylyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, oder CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
- R3: H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
- R4: NR6R7;
R6 und R7 unabhängig voneinander H, (C₁-C₆)-Alkyl, (C₃-C₆)-Cycloalkyl, Phenyl, wobei der Phenylring bis zu zweimal mit Cl, F, CN, CF₃, (C₁-C₃)-Alkyl, OH oder O-(C₁-C₆)-Alkyl substituiert sein kann; CO-(C₁-C₆)-Alkyl, CHO, CO-Phenyl, -NH₂, -N=C(CH₃)₂, -(pyrrolidin-1-yl), -(piperidin-1-yl), -(morpholin-4-yl), -(piperazin-1-yl), -(piperazin-4-methyl-1-yl),
oder
NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
sowie deren physiologisch verträgliche Salze

Bevorzugt sind Verbindungen der Formel I, worin bedeuten
- Y: eine direkte Bindung;
- X: CH₂;
- R₁, R1': unabhängig voneinander H, F, Cl, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂ , SO₂-(C₁-C₆)-Alkyl, SO₂-(CH₂)ₙ-Phenyl, wobei n = 0 - 6 sein kann und der Phenylrest mit F, Cl, Br, OH, CF₃, NO₂, CN, OCF₃, O-(C₁-C₆)-Alkyl, (C₁-C₆)-Alkyl oder NH₂ substituiert sein kann; NH₂, NH-(C₁-C₆)-Alkyl, N((C₁-C₆)-Alkyl)₂, NH(C₁-C₇)-Acyl, Phenyl, Biphenylyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 2-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂; 1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
- R3: H, (C₁-C₆)-Alkyl, F, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
- R4: NR6R7;
R6 und R7 unabhängig voneinander H, (C₁-C₆)-Alkyl, (C₃-C₆)-Cycloalkyl, Phenyl, wobei der Phenylring bis zu zweimal mit Cl, F, CN, CF₃, (C₁-C₃)-Alkyl, OH oder O-(C₁-C₆)-Alkyl substituiert sein kann; CO-(C₁-C₆)-Alkyl, CHO, CO-Phenyl, -NH₂, -N=C(CH₃)₂, -(pyrrolidin-1-yl), -(piperidin-1-yl), -(morpholin-4-yl), -(piperazin-1-yl), -(piperazin-4-methyl-1-yl),
oder
NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
sowie deren physiologisch verträgliche Salze

Besonders bevorzugt sind Verbindungen der Formel I, worin bedeuten
- Y: eine direkte Bindung;
- X: CH₂;
- R1, R1': unabhängig voneinander H, F, Cl, CF3, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-(C₁-C₆)-Alkyl, SO₂-(CH₂)ₙ-Phenyl, wobei n = 0 - 3 sein kann und der Phenylrest mit F, Cl, OH, CF₃, CN, OCF₃, O-(C₁-C₆)-Alkyl, (C₁-C₆)-Alkyl oder NH₂ substituiert sein kann;
NH₂, NH-(C₁-C₆)-Alkyl, N((C₁-C₆)-Alkyl)₂, NH(C₁-C₇)-Acyl, Phenyl, Biphenylyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 2fach substituiert sein können mit F, Cl, OH, CF₃, CN, OCF₃, O-(C₁-C₄)-Alkyl, (C₁-C₄)-Alkyl, NH₂, NH(C₁-C₄)-Alkyl, N((C₁-C₄)-Alkyl)₂, SO₂-CH₃, COOH, COO-(C₁-C₄-Alkyl, oder CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
- R2: H, (C₁-C₆)-Alkyl, (CH₂)ₙ-Phenyl, (CH₂)ₙ-Thienyl, (CH₂)ₙ-Pyridyl, (CH₂)ₙ-Furyl worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
- R3: H, F; (C₁-C₄)-Alkyl;
- R4: NR6R7;
R6 und R7 unabhängig voneinander H, (C₁-C₆)-Alkyl, (C₃-C₆)-Cycloalkyl, Phenyl, wobei der Phenylring bis zu zweimal mit Cl, F, CN, CF₃, (C₁-C₃)-Alkyl, OH oder O-(C₁-C₆)-Alkyl substituiert sein kann;
CO-(C₁-C₆)-Alkyl, CHO, CO-Phenyl, -NH₂, -N=C(CH₃)₂, -(pyrrolidin-1-yl), -(piperidin-1-yl), -(morpholin-4-yl), -(piperazin-1-yl), -(4-methylpiperazin-1-yl),
oder
NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkyl-, Alkenyl- und Alkinylreste in den Substituenten R1, R1', R2, R3, R4, R6 und R7 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Für medizinische Zwecke wird in besonders bevorzugter Weise das Chlorid verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht des vom Salz abgeleiteten Dihydrothiazolium-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

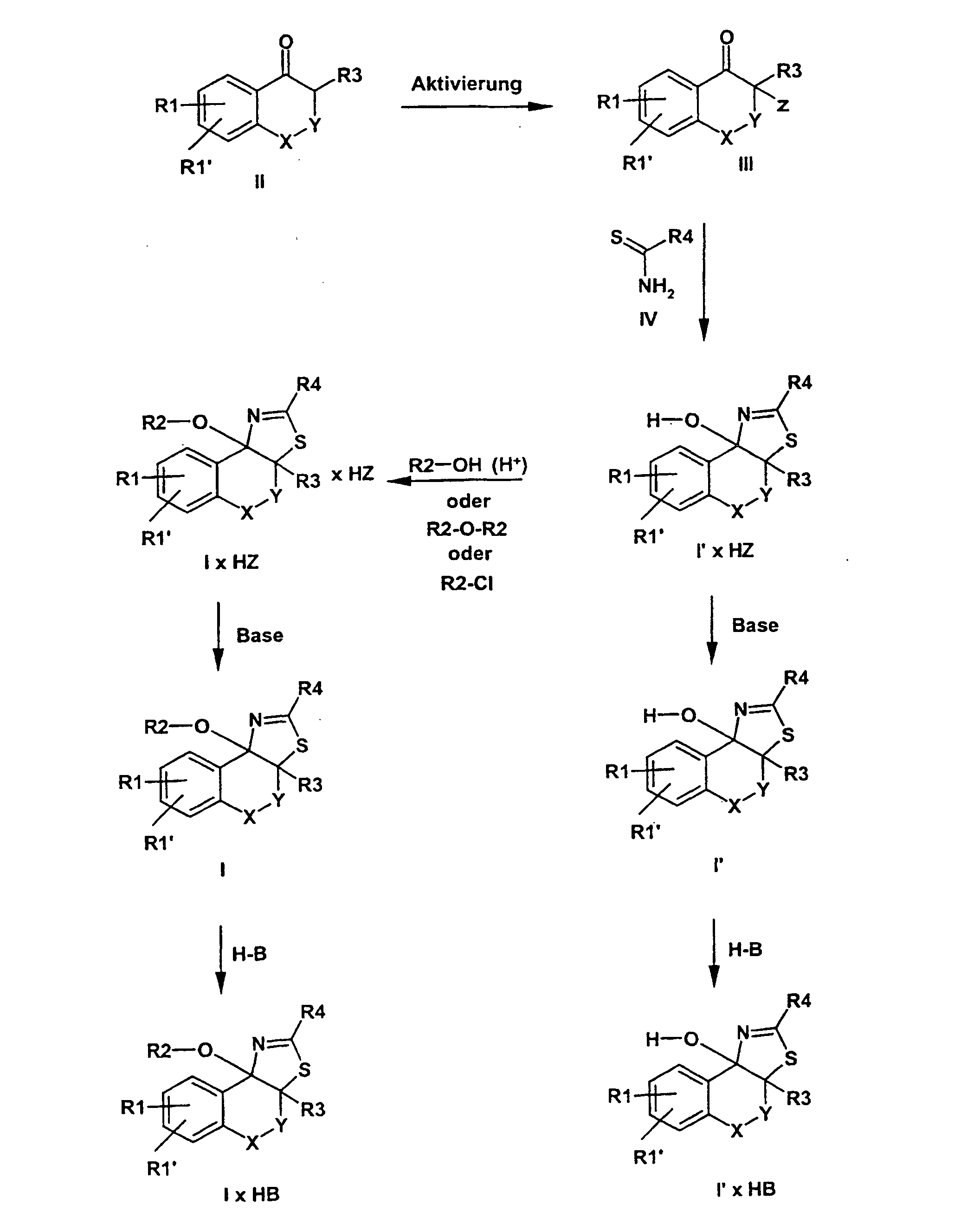

Dazu werden Verbindungen der allgemeinen Formel II, 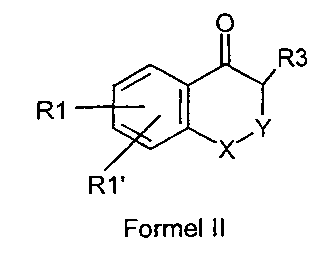 worin R1, R1', R3 und X und Y die angegebene Bedeutung besitzen, aktiviert und in eine Verbindung der Formel III überführt, worin Z für den Rest eines aktivierten Esters einer anorganischen oder organischen Säure steht.

Die Verbindungen der Formel III werden weiter mit Thioharnstoffen der Formel IV 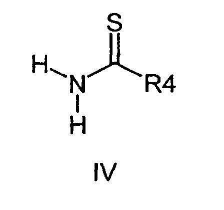 worin R4 die angegebene Bedeutung besitzt, zu Verbindungen der Formel I' x HZ bzw. I' umgesetzt, wobei gegebenenfalls die Verbindungen der allgemeinen Formel I' mit organischen oder anorganischen Säuren H-B in ihre Säureadditionssalze der Formel I' x HBoder erhaltene Salze der Formel I' x HZ mit organischen oder anorganischen Basen in die freien basischen Verbindungen der Formel I' überführt werden.

Als anorganische Säuren kommen beispielsweise in Betracht:
Halogenwasserstoffsäuren wie Chlorwasserstoffsäure und Bromwasserstoffsäure, sowie Schwefelsäure, Phosphorsäure und Amidosulfonsäure.

Als organische Säuren seien beispielsweise genannt: Ameisensäure, Essigsäure, Benzoesäure, p-Toluolsulfonsäure, Benzolsulfonsäure, Bernsteinsäure, Fumarsäure, Maleinsäure, Milchsäure, Weinsäure, Zitronensäure, L-Ascorbinsäure, Salizylsäure, Isäthionsäure, Methansulfonsäure, Trifluormethansulfonsäure, 1,2-Benzisothiazol-3(2H)-on, 6-Methyl-1,2,3-oxathiazin-4(3H)-on-2,2-dioxid.

Die oben beschriebene Verfahrensweise wird vorteilhaft so ausgeführt, daß man die Verbindungen III mit den Thioharnstoffen IV im molaren Verhältnis von 1:1 bis 1:1,5 umsetzt. Die Reaktion wird vorteilhaft in einem inerten Lösemittel, z.B. in polaren organischen Lösemitteln wie Dimethylformamid, Dimethylacetamid, N-Methyl-2-pyrrolidon, Dioxan, Tetrahydrofuran, Acetonitril, Nitromethan oder Diethylenglykoldimethylether durchgeführt. Als besonders vorteilhafte Lösemittel erweisen sich jedoch Essigsäuremethylester und Essigsäureethylester, kurzkettige Alkohole wie Methanol, Ethanol, Propanol, Isopropanol, sowie niedere Dialkylketone, wie z.B. Aceton, Butan-2-on oder Hexan-2-on. Auch Gemische der angeführten Reaktionsmedien können angewandt werden; so wie auch Gemische der aufgeführten Lösemittel mit Solventien, die für sich alleine genommen weniger geeignet sind, verwendet werden können, wie z.B. Gemische aus Methanol mit Benzol, Ethanol mit Toluol, Methanol mit Diethylether oder mit tert.Butylmethylether, Ethanol mit Tetrachlormethan, Aceton mit Chloroform, Dichlormethan oder 1,2-Dichlorethan, wobei das jeweils polarere Lösemittel zweckmäßigerweise im Überschuß verwendet werden soll. Die Reaktionspartner können im jeweiligen Reaktionsmedium suspendiert oder gelöst vorliegen. Grundsätzlich können die Reaktionspartner auch ohne Lösemittel umgesetzt werden, insbesondere dann, wenn das jeweilige Thioamid einen möglichst tiefen Schmelzpunkt hat. Die Reaktion verläuft nur wenig exotherm und kann zwischen -10°C und 150°C, bevorzugt zwischen 20°C und 50°C, durchgeführt werden. Als besonders günstig erwies sich in der Regel ein Temperaturbereich zwischen 0°C und 40°C.

Die Reaktionsdauer ist weitgehend von der Reaktionstemperatur abhängig und liegt zwischen 2 Minuten und 3 Tagen bei höheren bzw. niedrigeren Temperaturen. Im günstigen Temperaturbereich liegt die Reaktionsdauer im allgemeinen zwischen 5 Minuten und 48 Stunden.

Häufig scheiden sich die Verbindungen I' x HZ in Form ihrer Säureadditionssalze im Verlauf der Reaktion schwerlöslich ab, zwechmäßig wird nachträglich noch ein geeignetes Fällungsmittel zugesetzt. Als solches verwendet man z.B. Kohlenwasserstoffe wie Benzol, Toluol, Cyclohexan oder Heptan oder Tetrachlormethan; insbesondere erwiesen sich Essigsäurealkylester wie Essigsäureethylester oder Essigsäure-n-butylester oder Dialkylether wie Diethylether, Diisopropylether, Di-n-butylether oder tert.Butylmethylether als besonders geeignet. Bleibt nach Ende der Reaktion das Reaktionsgemisch in Lösung, so kann man die Salze der Verbindungen I' x HZ, ggf. nach Konzentrierung der Reaktionslösung, mit einem der genannten Fällungsmittel ausfällen. Ferner kann man die Lösung des Reaktionsgemisches auch vorteilhaft in die Lösung eines der genannten Fällungsmittel unter Rühren einfiltrieren. Da die Reaktion der Verbindungen III mit den Thioharnstoffen IV praktisch quantitativ abläuft, sind die erhaltenen Rohprodukte meistens bereits analytisch rein. Die Aufarbeitung des Reaktionsgemisches kann auch so durchgeführt werden, daß die Reaktionsmischung unter Zusatz einer organischen Base, wie z.B. Triethylamin oder Diisobutylamin oder Ammoniak oder Morpholin oder Piperidin oder 1,8-Diazabicyclo[5.4.0]undec-7-en alkalisch gestellt wird, und das Reaktionsrohprodukt nach Konzentrierung chromatographisch, z.B. über eine Kieselgelsäule, gereinigt wird. Als geeignete Elutionsmedien dafür erweisen sich z.B. Gemische von Essigsäureethylester mit Methanol, Gemische von Dichlormethan mit Methanol, Gemische von Toluol mit Methanol oder Essigsäureethylester oder Gemische von Essigsäurethylester mit Kohlenwasserstoffen wie Heptan. Erfolgt die Reinigung des Rohproduktes auf die zuletzt beschriebene Weise, kann aus der so gewonnenen reinen Base der Formel I' ein Säureadditionsprodukt der Formel I' x H-B so gewonnen werden, daß man die Base in einem organischen protischen Lösemittel wie Methanol, Ethanol, Propanol oder Isopropanol oder in einem organischen aprotischen Lösemittel wie Essigsäureethylester, Diethylether, Diisopropylether, tert.Butylmethylether, Dioxan, Tetrahydrofuran, Aceton oder Butan-2-on löst oder suspendiert und diese Mischung anschließend mit einer wenigstens äquimolaren Menge einer anorganischen Säure wie z.B. Chlorwasserstoffsäure, gelöst in einem inerten Lösemittel wie z.B. Diethylether oder Ethanol, oder einer anderen der weiter oben genannten anorganischen oder organischen Säuren versetzt.

Die Verbindungen der Formel I' können aus einem inerten, geeigneten Lösemittel wie z.B. Aceton, Butan-2-on, Acetonitril, Nitromethan umkristallisiert werden. Besonders vorteilhaft ist aber die Umfällung aus einem Lösemittel wie z.B. Dimethylformamid, Dimethylacetamid, Nitromethan, Acetonitril, vorzugsweise Aceton oder Essigsäureethylester.

Die Reaktion der Verbindungen der Formel III mit den Thioharnstoffen der Formel IV kann auch so durchgeführt werden, daß man zur Reaktionsmischung eine wenigstens äquimolare Menge einer Base, wie z.B. Triethylamin, zufügt und die so erhaltenen Verbindungen I' anschließend gegebenenfalls in ihre Säureadditionsprodukte I' x H-B überführt.

In den Verbindungen der Formel III kommen als Rest eines aktivierten Esters Z beispielsweise in Frage: Cl, Br, J, O-C(O)-(C₆H₄)-4-NO₂, O-SO₂-CH₃, O-SO₂-CF₃, O-SO₂-(C₆H₄)-4-CH₃, O-SO₂-C₆H₄.

Die Säureadditionsprodukte I' x HZ und I x HZ können durch Behandlung mit Basen zu den Verbindungen der allgemeinen Formel I' und I umgesetzt werden. Als Basen kommen beispielsweise Lösungen anorganischer Hydroxide, wie Lithium-, Natrium-, Kalium-, Calcium- oder Bariumhydroxid, Carbonate oder Hydrogencarbonate, wie Natrium- oder Kaliumcarbonat, Natrium- oder Kaliumhydrogencarbonat, Ammoniak und Amine, wie Triethylamin, Diisopropylamin, Dicyclohexylamin, Piperidin, Morpholin, Methyldicyclohexylamin in Frage.

Thioharnstoffe der allgemeinen Formel IV sind entweder kommerziell erhältlich oder können nach literaturbekannten Methoden hergestellt werden.

Die Verbindungen der Formel I x HZ bzw. I, wobei R2 (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl ist, und wobei n = 0 - 5 sein kann, lassen sich gewinnen, indem man entweder
aa) die Säureadditionssalze der Formel I' x HZ in einem Lösemittel der Formel R2-OH, wobei R2 die oben beschriebene Bedeutung hat, bei einer Temperatur von -20°C bis 100°C, vorzugsweise bei -5°C bis 50°C, 2 Stunden bis 4 Tage, vorzugsweise 4 Stunden bis 2 Tage, reagieren läßt
   oder
ab) die freien Basen der Formel I' in einem Lösemittel der Formel R2-OH, wobei R2 die oben beschriebene Bedeutung hat, mit äquimolaren, unterschüssigen oder katalytischen, vorzugsweise katalytischen Mengen einer anorganischen oder organischen Säure, wie sie weiter oben beschrieben sind, oder unter Zugabe eines sauren Ionenaustauschers bei einer Temperatur von -20°C bis 100°C, vorzugsweise bei -5°C bis 50°C, 2 Stunden bis 4 Tage, vorzugsweise 4 Stunden bis 2 Tage, umsetzt
   oder
ac) die Umsetzungen nach aa) und ab) in einem inerten aprotischem Lösemittel wie Dichlormethan, Chloroform, 1,2-Dichlorethan, Heptan, Benzol, Toluol, Acetonitril, Nitromethan, Dioxan, Tetrahydrofuran, Ethylenglykoldimethylether, Diethylether, Diisopropylether, tert.Butylmethylether, Aceton, Butan-2-on oder Essigsäureniederalkylester, wie z.B. Essigsäureethylester, durch Zugabe von 1 bis 5, vorzugsweise 1,5-2 Äquivalenten einer Verbindung der Formel R2-OH durchführt
   oder
ad) Verbindungen der Formel I' in einem polaren aprotischen Lösemittel, wie z.B. Tetrahydrofuran, Dioxan, Ethylenglykoldimethylether, Nitromethan, Acetonitril oder Dimethylformamid, Dimethylacetamid oder N-Methyl-2-pyrrolidon, mit Hilfe einer Base, wie z.B. Natriumhydrid, Lithiumdiisopropylamid, KOH oder Kaliumcarbonat, in ihr Alkoholat überführt und dieses dann unter Zugabe eines Alkylierungsmittels der Formel R2-W, wobei W = Chlor, Brom, Jod, O-C(O)-CH₃, O-C(O)-CF₃, O-C(O)-C₆H₄-4-NO₂, O-SO₂-CH₃, O-SO₂-CF₃, O-SO₂-C₆H₄-4-CH₃, O-SO₂-C₆H₄-4-NO₂, bei -20 bis 150°C, bevorzugt bei -15 bis 50°C, für 10 Minuten bis 2 Tage, bevorzugt für 20 Minuten bis 12 Stunden, reagieren läßt. Verbindungen der Formel I x HZ oder I mit R2 = C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann, lassen sich gewinnen, indem man entweder
ba) wie unter aa) - ac) beschrieben vorgeht, mit dem Unterschied, daß man statt eines Alkohols R2-OH eine Säure R2-OH einsetzt, und man 1 bis 2 Äquivalente der Säure R2-OH, vorzugsweise 1,5 Äquivalente der Säure R2-OH einsetzt und auf den Zusatz des unter aa) - ac) beschriebenen sauren anorganischen oder organischen Katalysators verzichtet, den sauren Kationenaustauscher jedoch vorteilhaft einsetzt
   oder
bb) eine Verbindung der Formel I' mit einer Säure der Formel R2-OH im Sinne einer Mitsunobu Reaktion (O. Mitsunobu, Synthesis 1981, 1) zu einer Verbindung der Formel I umsetzt
   oder
bc) ein Carbonsäurechlorid der Formel R2-Cl oder ein Carbonsäureanhydrid der Formel R2-O-R2 mit einer Verbindung der Formel I' im Sinne einer Veresterung eines Alkohols (Houben-Weyl, Methoden der Organischen Chemie, Georg Thieme Verlag Stuttgart, Band E5, S. 656-715) umsetzt.

Die nachfolgend aufgeführten Beispiele dienen zur Erläuterung der Erfindung, ohne diese jedoch einzuschränken. Die gemessenen Fusions-, bzw. Zersetzungspunkte (Fp.) wurden nicht korrigiert und sind generell von der Aufheizgeschwindigkeit abhängig.

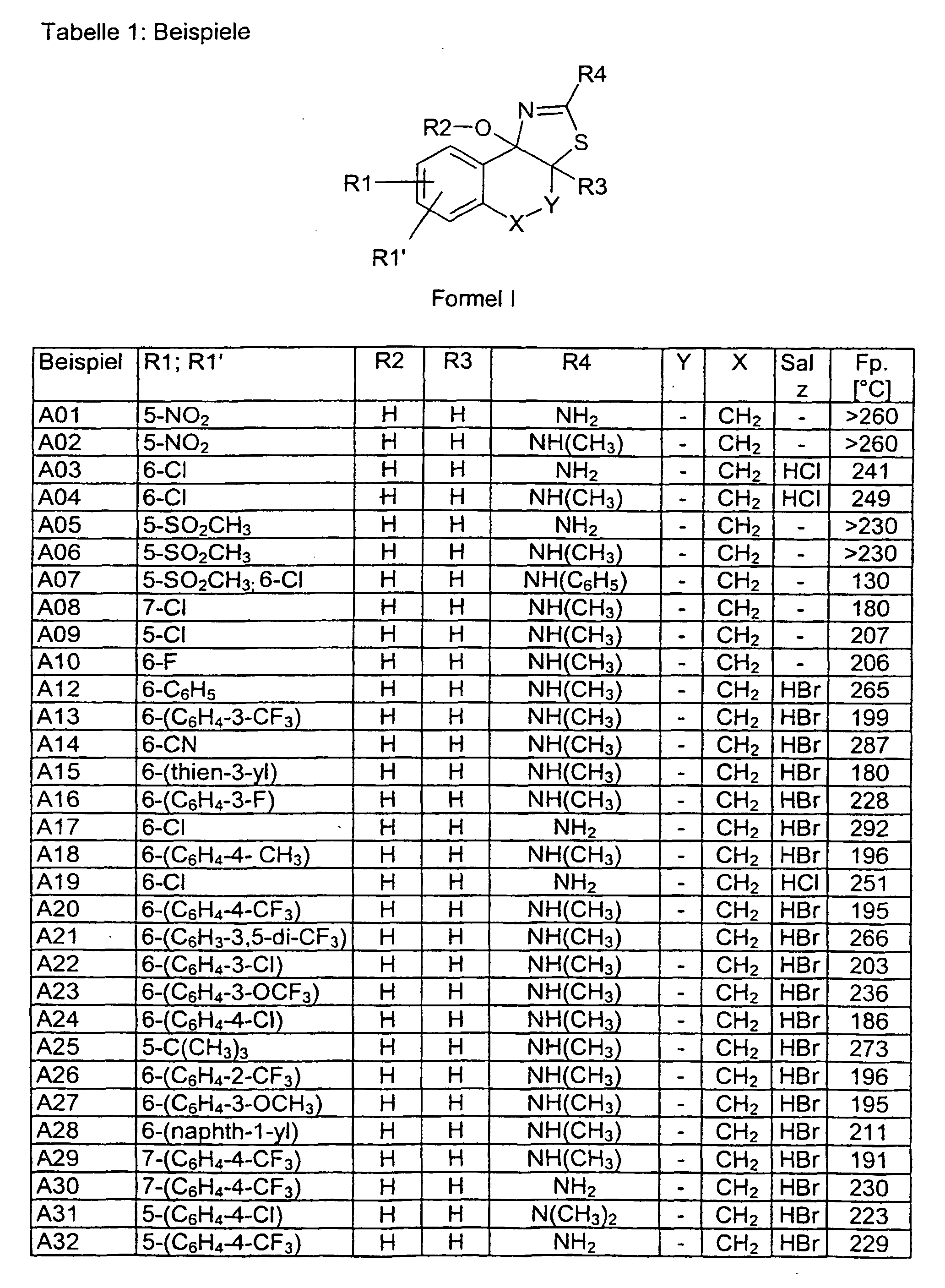

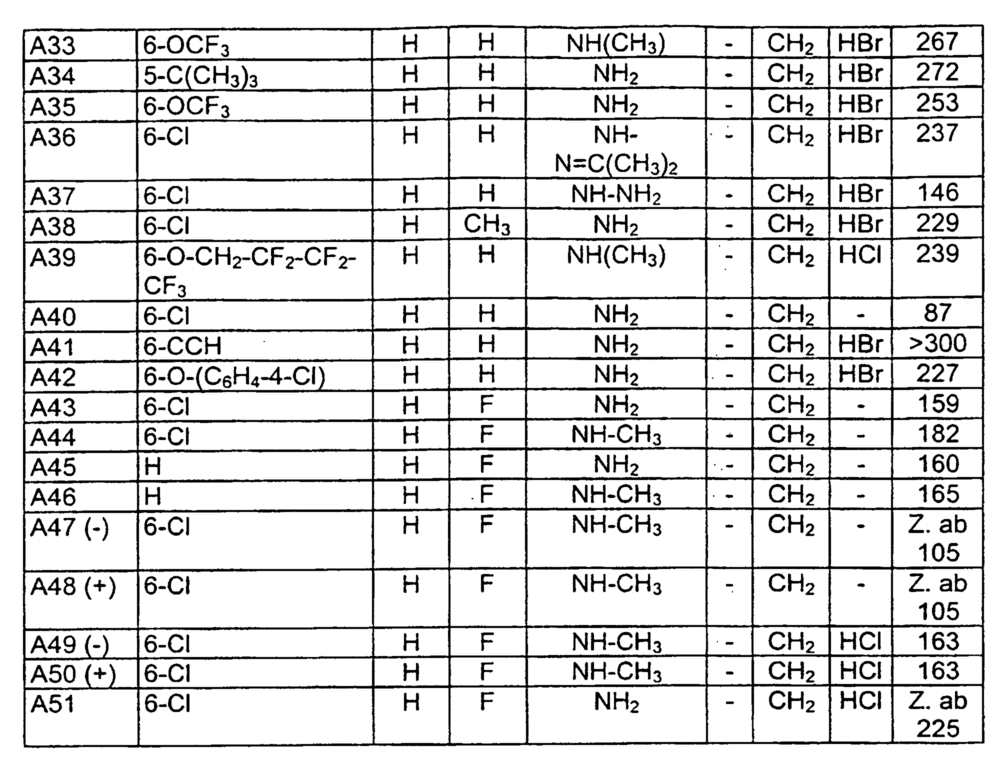

Die Verbindungen der Formel I zeichnen sich durch günstige Wirkungen auf den Fettstoffwechsel aus, insbesondere sind sie als Anorektika geeignet. Die Verbindungen können allein oder in Kombination mit weiteren anorektischen Wirkstoffen eingesetzt werden. Solche weiteren anorektischen Wirkstoffe werden z.B. in der Roten Liste, Kapitel 01 unter Abmagerungsmittel/Appetitzügler genannt. Die Verbindungen eignen sich zur Prophylaxe sowie insbesondere zur Behandlung von Obesitas. Die Verbindungen eignen sich weiterhin zur Prophylaxe sowie insbesondere zur Behandlung von Typ II Diabetes.

### Die Wirksamkeit der Verbindungen wurde wie folgt getestet:

### Biologisches Prüfmodell:

Die Prüfung der anorektischen Wirkung erfolgte an männlichen NMRI Mäusen. Nach 24stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit dem unbehandelter Kontrolltieren verglichen.

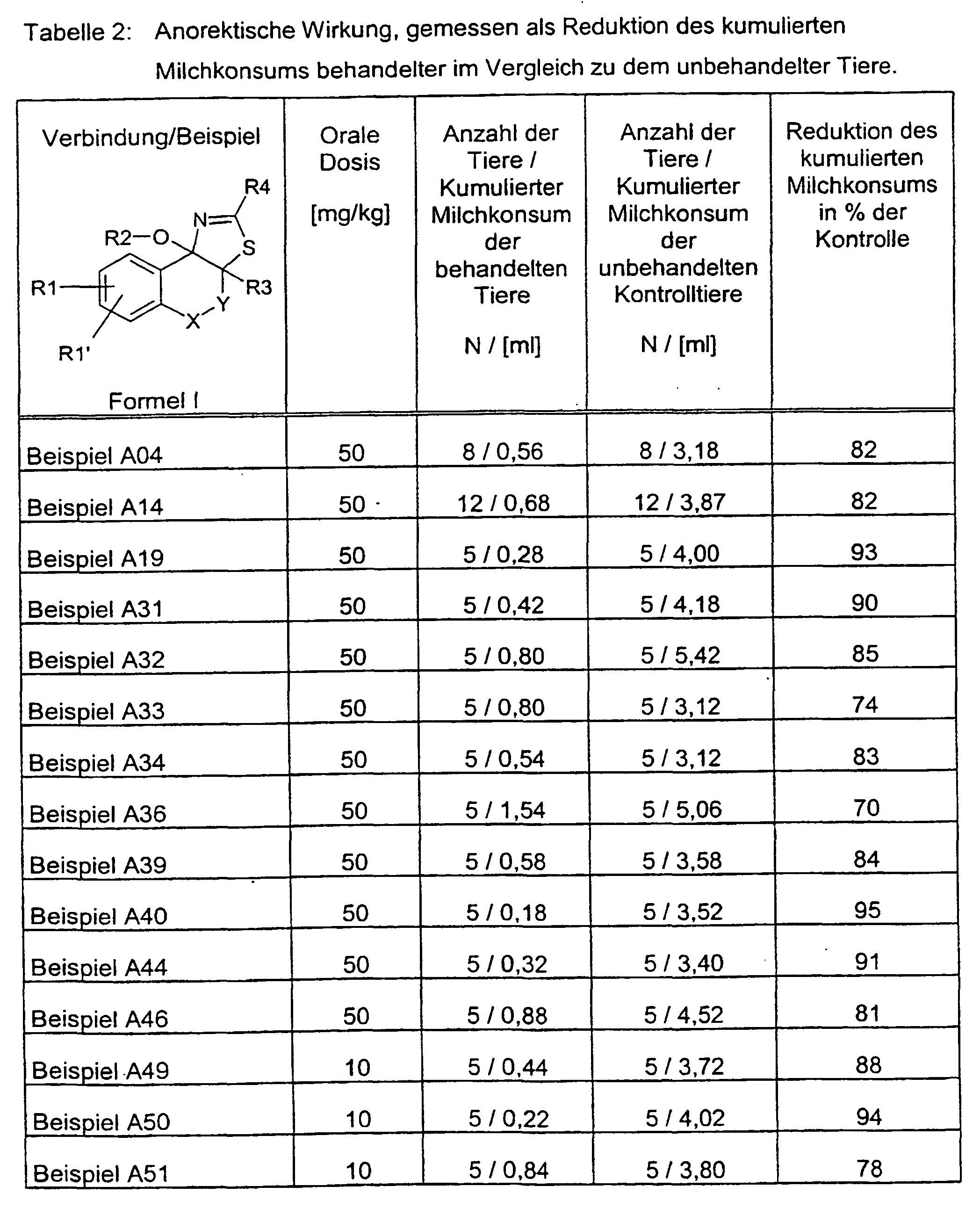

Aus der Tabelle ist abzulesen, daß die Verbindungen der Formel I eine sehr gute anorektische Wirkung zeigen.

Nachfolgend wird die Herstellung einiger Beispiele detailliert beschrieben, die übrigen Verbindungen der Formel I wurden analog erhalten:

### Ausführungsbeispiel 1:

2-Amino-5-nitro-8,8a-dihydro-indeno[1,2-d]thiazol-3a-ol (Verbindung des Beispiels 1):

### a) 2-Brom-6-nitro-indan-1-on:

7.1 g (0.04 mol) 5-Nitroindan-1-on werden in 135 ml Eisessig gelöst und mit 1.12 ml 48 proz. Bromwaserstofflösung versetzt. Anschließend gibt man zur Lösung tropfenweise bei Raumtemp. 2.06 ml Brom in 40 ml Eisessig und rührt 2 h nach. Man gießt das Reaktionsgemisch auf Eiswasser, extrahiert mit Dichlormethan, trocknet und befreit die org. Phase i. Vak. vom Lösemittel. Der Rückstand wird durch Säulenfiltration (Kieselgel; Dichlormethan) gereinigt. Man erhält das 2-Brom-6-nitro-indan-1-on mit dem Schmelzpunkt 113°C.

### b) 2-Amino-5-nitro-8,8a-dihydro-indeno[1,2-d]thiazol-3a-ol:

1.28 g (5 mmol) 2-Brom-6-nitro-indan-1-on werden in 30 ml Aceton gelöst und unter Rühren mit 530 mg Thioharnstoff in 10 ml Aceton versetzt. Aus der zunächst klaren Lösung kristallisiert nach wenigen Minuten das Hydrobromid des 2-Amino-5-nitro-8,8a-dihydro-indeno[1,2-d]thiazol-3a-ols aus. Man rührt 1 h bei Raumtemp. nach, saugt ab und wäscht mit wenig Aceton. Das lufttrockene Hydrobromid wird in ca. 10 ml Methanol gelöst, mit 0.7 ml Triethylamin versetzt und 15 min gerührt. Dann gibt man 50 ml Wasser dazu und rührt 1 h bei Raumtemp. Die gebildeten Kristalle werden abgesaugt und mit wenig kaltem Wasser nachgewaschen. Man erhält 2-Amino-5-nitro-8,8a-dihydro-indeno[1,2-d]thiazol-3a-ol mit einem Schmelzpunkt über 250°C.

### Ausführungsbeispiel 2:

### 3a-Hydroxy-2-methylamino-8,8a-dihydro-3aH-indeno[1,2-d]thiazol-6-carbonitril Hydrobromid (Verbindung des Beispiels 14):

### a) 1-Oxo-indan-5-carbonitril:

9,5 g 5-Brom-indan-1-on und 4,93 g CuCN werden in 10 ml Dimethylformamid suspendiert und 4 Stunden unter Rückfluß gekocht. Zur abgekühlten, dunkelbraunen, viskosen Suspension wird unter Rühren eine Lösung von 18 g Eisen-III-chlorid in 5 ml konz. Salzsäure mit 30 ml Wasser zugetropft und anschließend für 30 Minuten bei 70°C gerührt. Die Reaktionsmischung wird dreimal mit 50 ml Toluol ausgeschüttelt, und die vereinigten organischen Phasen werden mit 50 ml 2N Salzsäure und 50 ml 2 N Natronlauge ausgeschüttelt und dann mit Wasser neutral gewaschen. Der Toluolextrakt wird über Magnesiumsulfat getrocknet, im Vakuum eingeengt, und der Rückstand wird aus n-Heptan umkristallisiert. Man erhält 1-Oxo-indan-5-carbonitril mit dem Schmelzpunkt 123 - 125°C.

### b) 2-Brom-1-oxo-indan-5-carbonitril:

3,93 g 1-Oxo-indan-5-carbonitril werden in 40 ml Eisessig gelöst, mit 0,1 ml Bromwasserstoffsäure (48%ig in Wasser) versetzt und bei Raumtemperatur tropfenweise mit einer Lösung von 1,34 ml Brom in 8 ml Essigsäure versetzt. Die Reaktionsmischung wird 3 h bei Raumtemperatur gerührt, anschließend auf eine Mischung aus 20 g Eis mit 40 g Wasser gegeben, mit 0,5 g Natriumhydrogensulfit versetzt und zweimal mit jeweils 50 ml Dichlormethan ausgeschüttelt. Die organischen Phase wird mit 50 ml Wasser gewaschen, über Magnesiumsulfat getrocknet, im Vakuum eingeengt und über Kieselgel mit Toluol / Essigsäureethylester 25 / 1 chromatographiert. Man erhält 2-Brom-1-oxo-indan-5-carbonitril mit dem Schmelzpunkt 115 - 118°C.

### c) 3a-Hydroxy-2-methylamino-8,8a-dihydro-3aH-indeno[1,2-d]thiazol-6-carbonitril Hydrobromid:

236 mg 2-Brom-1-oxo-indan-5-carbonitril werden in 10 ml Aceton gelöst und bei 0°C mit 135 mg N-Methylthioharnstoff versetzt. Die Mischung wird 3 h bei Raumtemperatur und 90 Minuten bei Eisbadtemperatur gerührt. Der Niederschlag wird abgesaugt, mit Aceton gewaschen und im Vakuum getrocknet. Man erhält das Hydrobromid des 3a-Hydroxy-2-methylamino-8,8a-dihydro-3aH-indeno[1,2-d]thiazol-6-carbonitrils mit dem Schmelzpunkt 287 - 288°C.

### Ausführungsbeispiel 3:

### 2-Amino-5-(4-trifluormethyl-phenyl)-8,8a-dihydro-indeno[1,2-d]thiazol-3a-ol Hydrobromid (Verbindung des Beispiels 32):

### a) 6-(4-Trifluormethyl-phenyl)-indan-1-on:

6,33 g 6-Brom-indan-1-on werden mit 5,7 g 4-Trifluormethylbenzolboronsäure und 6,36 g Natriumcarbonat in einer Mischung aus 100 ml trockenem Toluol mit 20 ml trockenem Ethanol und 20 ml Wasser suspendiert und unter Argon nach Zugabe von 338 mg Palladium-II-acetat und 787 mg Triphenylphosphin 4 h unter Rückfluß gekocht. Die Reaktionsmischung wird abgekühlt, der Ethanolanteil im Vakuum entfernt, der Rückstand mit 40 ml 0,5 N Natronlauge 10 Minuten gerührt und über eine Klärschicht abgesaugt. Die resultierende Lösung wird dreimal mit 50 ml Wasser und einmal mit 50 ml ges. Kochsalzlösung gewaschen, über Magnesiumsulfat getrocknet, im Vakuum eingeengt und über Kieselgel mit Dichlormethan / Heptan 3 / 1 chromatographiert. Man erhält 6-(4-Trifluormethyl-phenyl)-indan-1-on, welches ohne weitere Reinigung in die nächste Stufe eingesetzt wird.

### b) 2-Brom-6-(4-trifluormethyl-phenyl)-indan-1-on:

Die Bromierung wird wie im Ausführungsbeispiel 2b beschrieben durchgeführt und liefert 2-Brom-6-(4-trifluormethyl-phenyl)-indan-1-on mit dem Schmelzpunkt 105°C.

### c) 2-Amino-5-(4-trifluormethyl-phenyl)-8,8a-dihydro-indeno[1,2-d]thiazol-3a-ol Hydrobromid:

1,06 g 2-Brom-6-(4-trifluormethyl-phenyl)-indan-1-on werden mit 266 mg Thioharnstoff in 10 ml trockenem Aceton gelöst und 4 h bei Eisbadtemperatur gerührt. Der Niederschlag wird abgesaugt, mit Aceton gewaschen und im Vakuum getrocknet. Man erhält das Hydrobromid des 2-Amino-5-(4-trifluormethyl-phenyl)-8,8a-dihydro-indeno[1,2-d]thiazol-3a-ols mit dem Schmelzpunkt 228 - 230°C.

### Ausführungsbeispiel 4:

### 2-Amino-6-chlor-8a-methyl-8,8a-dihydro-indeno[1,2-d]thiazol-3a-ol Hydrobromid (Verbindung des Beispiels 38):

20 g 5-Chlor-indan-1-on werden in 600 ml trockenem Toluol gelöst, mit 27,4 ml N,N-Dimethylhydrazin und 1 g p-Toluolsulfonsäure versetzt und 4 h am Wasserabscheider gekocht. Die Reaktionsmischung wird im Vakuum eingeengt, der Rückstand in Essigsäureethylester gelöst und mit Natriumhydrogencarbonat lösung und anschließend mit Wasser gewaschen. Die organische Phase wird über Natriumsulfat getrocknet, im Vakuum eingeengt und der Rückstand über Kieselgel mit Essigsäureethylester / Heptan 1 / 2 chromatographiert. Man erhält N'-(5-Chlor-indan-1-yliden)-N,N-dimethyl-hydrazin als Öl.

Zu 300 ml trockenem Tetrahydrofuran (THF) werden unter Argon 33,1 ml einer 2M-Lösung von Lithiumdiisopropylamid in THF/Heptan/Ethylbenzol zugetropft. Bei -70°C werden zu dieser Lösung 12 g N'-(5-Chlor-indan-1-yliden)-N,N-dimethyl-hydrazin, gelöst in 100 ml trockenem THF, zugetropft. Nachdem die Reaktionsmischung 1 h bei -70°C und 3 weitere h bei -40°C gerührt hat, werden 3,882 ml Methyljodid zugetropft und die Mischung über Nacht weitergerührt. Zur auf Raumtemperatur erwärmten Reaktionsmischung werden 200 ml Wasser zugetropft; Das THF wird im Vakuum entfernt und der Rückstand wird mit Essigsäureethylester extrahiert. Der Essigesterextrakt wird mit Aktivkohle behandelt, über Natriumsulfat getrocknet, filtriert, im Vakuum eingeengt und über Kieselgel mit Essigsäureethylester / Heptan 1 / 2 chromatographiert. Man erhält N'-(5-Chlor-2-methyl-indan-1-yliden)-N,N-dimethyl-hydrazin als ein Öl.

5,5 g N'-(5-Chlor-2-methyl-indan-1-yliden)-N,N-dimethyl-hydrazin werden in 100 ml 2 N Schwefelsäure emulgiert und 4 h unter Rückfluß gekocht. Die abgekühlte Reaktionsmischung wird vorsichtig mit Essigsäureethylester extrahiert; der organische Extrakt wird mit ges. Natriumhydrogencarbonatlösung mehrmals ausgeschüttelt, über Natriumsulfat getrocknet, filtriert, im Vakuum getrocknet und über Kieselgel mit Essigsäureethylester / Heptan 1 / 5 chromatographiert. Man erhält 5-Chlor-2-methyl-indan-1-on als ein Öl.

Die Bromierung des 5-Chlor-2-methyl-indan-1-ons wird wie im Ausführungsbeispiel 2b) beschrieben durchgeführt und liefert 2-Brom-5-chlor-2-methyl-indan-1-on als ein Öl, welches ohne weitere Reinigung in die nächste Stufe eingesetzt wird.

1,5 g 2-Brom-5-chlor-2-methyl-indan-1-on werden zusammen mit 460 mg Thioharnstoff in 50 ml trockenem Aceton gelöst und 8 h unter Rückfluß gekocht. Der Niederschlag wird abgesaugt, mit Aceton gewaschen und in Vakuum getrocknet. Man erhält 2-Amino-6-chlor-8a-methyl-8,8a-dihydro-indeno[1,2-d]thiazol-3a-ol Hydrobromid, welches bei 229°C unter Zersetzung schmilzt.

### Ausführungsbeispiel 5:

### 6-(2,2,3,3,4,4,4-Heptafluor-butoxy)-2-methylamino-8,8a-dihydro-indeno[1,2-d]thiazol-3a-ol Hydrochlorid (Verbindung des Beispiels 39):

### a) 6-(2,2,3,3,4,4,4-Heptafluor-butoxy)-2-methylamino-8,8a-dihydro-indeno[1,2-d]thiazol-3a-ol Hydrobromid

erhält man durch Umsetzung von 1,7g 2 Brom-5-(2,2,3,3,4,4,4-heptafluor-butoxy)-indan-1-on mit 0,31 g N-Methylthioharnstoff in 30 ml Ethylacetat und 24 Stunden Rührung bei Raumtemperatur als farblosen kristallinen Niederschlag. Schmp. 280-282 °C (Zersetzung)

### b) 6-(2,2,3,3,4,4,4-Heptafluor-butoxy)-2-methylamino-8,8a-dihydro-indeno[1,2-d]thiazol-3a-ol

erhält man durch Behandlung von 0,9g 6-(2,2,3,3,4,4,4-Heptafluor-butoxy)-2-methylamino-8,8a-dihydro-indeno[1,2-d]thiazol-3a-ol Hydrobromid mit 0.72g Triethylamin in 30 ml Ethanol. Nach Stehenlassen über Nacht filtriert man den kristallinen Niederschlag ab und wäscht mit Wasser mehrmals nach. Farblose Kristalle, Schmp. 178-180°C.

### c) 6-(2,2,3,3,4,4,4-Heptafluor-butoxy)-2-methylamino-8,8a-dihydro-indeno[1,2-d]thiazol-3a-ol Hydrochlorid

erhält man durch Zugabe von etherischer Lösung Chlorwasserstoffgas zu einer Lösung von 0.6 g 6-(2,2,3,3,4,4,4-Heptafluor-butoxy)-2-methylamino-8,8a-dihydro-indeno[1,2-d]thiazol-3a-ol in 20 ml Ethylacetat. Man rührt etwa einen Tag bei Raumtemperatur und filtriert den farblosen kristallinen Niederschlag ab. Schmp. 238 - 240°C

### Ausführungsbeispiel 6:

### 2-Amino-6-chlor-8a-fluor-8,8a-dihydro-indeno[1,2-d]thiazol-3a-ol (Verbindung des Beispiels 43):

### a) 5-Chlor-2-fluor-indan-1-on:

Zu einer Lösung von 5,24 ml Diisopropylamin in 60 ml trockenem Tetrahydrofuran werden bei einer Temperatur von < -50°C 25 ml einer 1,6 molaren Lösung von n-Butyllithium in n-Hexan langsam zugetropft; die Mischung wird anschließend noch 10 Minuten bei -50°C gerührt. Sodann wird eine Lösung von 6,33 g 5-Chlor-indan-1-on in 60 ml trockenem Tetrahydrofuran langsam zugegeben und die Mischung weitere 20 Minuten bei -50°C gerührt. Schließlich werden 11,4 g N-Fluordibenzolsulfimid, gelöst in 60 ml trockenem Tetrahydrofuran, zugetropft. Unter Rühren läßt man die Mischung sich innerhalb von 2 Stunden auf 0°C erwärmen, tropft 120 ml einer ges. Natriumhydrogencarbonatlösung zu, destilliert das Tetrahydrofuran im Vakuum ab und schüttelt den Rückstand zweimal mit 150 ml Essigsäureethylester aus. Die organische Phase wird mit Wasser und ges. Kochsalzlösung gewaschen, über Magnesiumsulfat getrocknet, eingeengt und über Kieselgel mit Diisopropylether / n-Heptan 1/1 chromatographisch gereinigt. Neben 5-Chlor-2,2-difluor-indan-1-on erhält man 5-Chlor-2-fluor-indan-1-on mit dem Schmelzpunkt 102 - 104°C.

### b) 5-Chlor-2-brom-2-fluor-indan-1-on:

Die Bromierung des 5-Chlor-2-fluor-indan-1-ons erfolgt wie im Ausführungsbeispiel 2b) beschrieben und liefert 5-Chlor-2-brom-2-fluor-indan-1-on mit dem Schmelzpunkt 104 - 105°C.

### c) 2-Amino-6-chlor-8a-fluor-8,8a-dihydro-indeno[1,2-d]thiazol-3a-ol:

263 mg 5-Chlor-2-brom-2-fluor-indan-1-on und 152 mg Thioharnstoff werden in 2,5 ml trockenem Dimethylsulfoxid gelöst und 8 h bei 50°C gerührt. Die Reaktionsmischung wird mit einem Überschuß Triethylamin versetzt und im Vakuum eingeengt. Der Rückstand wird über Kieselgel mit Essigsäureethyl ester als Elutionsmittel chromatographisch gereinigt. Man erhält 2-Amino-6-chlor-8a-fluor-8,8a-dihydro-indeno[1,2-d]thiazol-3a-ol mit dem Schmelzpunkt 159°C.

## Patentansprüche

1. Verbindungen der Formel I, 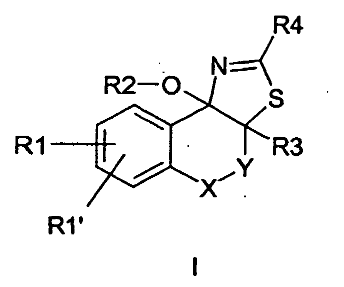 worin bedeuten
Y eine direkte Bindung, -CH₂-, -CH₂-CH₂-;
X CH₂, CH(CH₃), CH(C₂H₅), CH(C₃H₇), CH(C₆H₅);
R1, R1' unabhängig voneinander H, F, Cl, Br, J, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl oder NH₂ substituiert sein kann; NH₂, NH-(C₁-C₆)-Alkyl, N((C₁-C₆)-Alkyl)₂, NH(C₁-C₇)-Acyl, Phenyl, Biphenylyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 3-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, oder CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
R3 H, (C₁-C₆)-Alkyl, F, CN, N₃, O-(C₁-C₆)-Alkyl, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH_{3;}
R4 NR6R7;
R6 und R7 unabhängig voneinander H, (C₁-C₆)-Alkyl, (C₃-C₆)-Cycloalkyl, Phenyl, wobei der Phenylring bis zu zweimal mit Cl, F, CN, CF₃, (C₁-C₃)-Alkyl, OH oder O-(C₁-C₆)-Alkyl substituiert sein kann; CO-(C₁-C₆)-Alkyl, CHO, CO-Phenyl, -NH₂, -N=C(CH₃)₂, -(pyrrolidin-1-yl), -(piperidin-1-yl), -(morpholin-4-yl), -(piperazin-1-yl), -(4-methylpiperazin-1-yl),
oder
NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
sowie deren physiologisch verträgliche Salze.

2. Verbindungen der Formel I, gemäß Anspruch 1, **dadurch gekennzeichnet, daß** darin bedeuten
Y eine direkte Bindung;
X CH₂;
R1, R1' unabhängig voneinander H, F, Cl, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann; SO₂-NH₂, SO₂NH(C₁-C₆)-Alkyl, SO₂N[(C₁-C₆)-Alkyl]₂ , SO₂-(C₁-C₆)-Alkyl, SO₂-(CH₂)ₙ-Phenyl, wobei n = 0 - 6 sein kann und der Phenylrest mit F, Cl, Br, OH, CF₃, NO₂, CN, OCF₃, O-(C₁-C₆)-Alkyl, (C₁-C₆)-Alkyl oder NH₂ substituiert sein kann; NH₂, NH-(C₁-C₆)-Alkyl, N((C₁-C₆)-Alkyl)₂, NH(C₁-C₇)-Acyl, Phenyl, Biphenylyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 2-fach substituiert sein können mit F, Cl, Br, J, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl oder CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (C₃-C₆)-Cycloalkyl, (CH₂)ₙ-Phenyl, (CH₂)ₙ-Thienyl, (CH₂)ₙ-Pyridyl, (CH₂)ₙ-Furyl, C(O)-(C₁-C₆)-Alkyl, C(O)-(C₃-C₆)-Cycloalkyl, C(O)-(CH₂)ₙ-Phenyl, C(O)-(CH₂)ₙ-Thienyl, C(O)-(CH₂)ₙ-Pyridyl, C(O)-(CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
R3 H, (C₁-C₆)-Alkyl, F, (CH₂)ₙ-Phenyl, (CH₂)ₙ-Thienyl, (CH₂)ₙ-Pyridyl, (CH₂)ₙ-Furyl, wobei n = 0 - 5 sein kann und worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann, (C₂-C₆)-Alkinyl, (C₂-C₆)-Alkenyl, C(O)OCH₃, C(O)OCH₂CH₃, C(O)OH, C(O)NH₂, C(O)NHCH₃, C(O)N(CH₃)₂, OC(O)CH₃;
R4 NR6R7;
R6 und R7 unabhängig voneinander H, (C₁-C₆)-Alkyl, (C₃-C₆)-Cycloalkyl, Phenyl, wobei der Phenylring bis zu zweimal mit Cl, F, CN, CF₃, (C₁-C₃)-Alkyl, OH oder O-(C₁-C₆)-Alkyl substituiert sein kann; CO-(C₁-C₆)-Alkyl, CHO, CO-Phenyl, -NH₂, -N=C(CH₃)₂, -(pyrrolidin-1-yl), -(piperidin-1-yl), -(morpholin-4-yl), -(piperazin-1-yl), -(4-methylpiperazin-1-yl),
oder
NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
sowie deren physiologisch verträgliche Salze.

3. Verbindungen der Formel I, gemäß Anspruch 1 oder 2, **dadurch gekennzeichnet, daß** darin bedeuten
Y eine direkte Bindung;
X CH₂;
R1, R1' unabhängig voneinander H, F, Cl, CF3, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können, oder ein Wasserstoff durch OH, OC(O)CH₃, OC(O)H, O-CH₂-Ph, NH₂, NH-CO-CH₃ oder N(COOCH₂Ph)₂ ersetzt sein kann; SO₂-(C₁-C₆)-Alkyl, SO₂-(CH₂)ₙ-Phenyl, wobei n = 0 - 3 sein kann und der Phenylrest mit F, Cl, , OH, CF₃, CN, OCF₃, O-(C₁-C₆)-Alkyl, (C₁-C₆)-Alkyl oder NH₂ substituiert sein kann;
NH₂, NH-(C₁-C₆)-Alkyl, N((C₁-C₆)-Alkyl)₂, NH(C₁-C₇)-Acyl, Phenyl, Biphenylyl, O-(CH₂)ₙ-Phenyl, wobei n = 0 - 6 sein kann, 1- oder 2-Naphthyl, 2-, 3- oder 4-Pyridyl, 2- oder 3-Furanyl oder 2- oder 3-Thienyl, wobei die Phenyl-, Biphenylyl-, Naphthyl-, Pyridyl-, Furanyl- oder Thienylringe jeweils ein bis 2fach substituiert sein können mit F, Cl, OH, CF₃, CN, OCF₃, O-(C₁-C₄)-Alkyl, (C₁-C₄)-Alkyl, NH₂, NH(C₁-C₄)-Alkyl, N((C₁-C₄)-Alkyl)₂, SO₂-CH₃, COOH, COO-(C₁-C₄-Alkyl, oder CONH₂;
1,2,3-Triazol-5-yl, wobei der Triazolring in 1-, 2- oder 3-Stellung mit Methyl oder Benzyl substituiert sein kann;
Tetrazol-5-yl, wobei der Tetrazolring in 1- oder 2-Stellung mit Methyl oder Benzyl substituiert sein kann;
R2 H, (C₁-C₆)-Alkyl, (CH₂)ₙ-Phenyl, (CH₂)ₙ-Thienyl, (CH₂)ₙ-Pyridyl, (CH₂)ₙ-Furyl worin Phenyl, Thienyl, Pyridyl, Furyl jeweils bis zu zweimal mit Cl, F, CN, CF₃, (C₁-C₃)-Alkyl, OH oder O-(C₁-C₆)-Alkyl substituiert sein kann;
R3 H, F; (C₁-C₄)-Alkyl;
R4 NR6R7;
R6 und R7 unabhängig voneinander H, (C₁-C₆)-Alkyl, (C₃-C₆)-Cycloalkyl, Phenyl, wobei der Phenylring bis zu zweimal mit Cl, F, CN, CF₃, (C₁-C₃)-Alkyl, OH oder O-(C₁-C₆)-Alkyl substituiert sein kann;
CO-(C₁-C₆)-Alkyl, CHO, CO-Phenyl, -NH₂, -N=C(CH₃)₂, -(pyrrolidin-1-yl), -(piperidin-1-yl), -(morpholin-4-yl), -(piperazin-1-yl), -(4-methylpiperazin-1-yl),
oder
NR6R7 stellt einen Ring wie Pyrrolidin, Piperidin, Morpholin, Piperazin, N-4-Methyl-piperazin-1-yl, N-4-Benzyl-piperazin-1-yl, Phthalimidyl dar;
sowie deren physiologisch verträgliche Salze.

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und ein oder mehrere anorektische Wirkstoffe.

6. Verbindungen gemäß einem oder mehreren der Ansprüche 1 bis 3 zur Anwendung als Medikament zur Prophylaxe oder Behandlung der Obesitas.

7. Verbindungen gemäß einem oder mehreren der Ansprüche 1 bis 3 zur Anwendung als Medikament zur Prophylaxe oder Behandlung des Typ II Diabetes.

8. Verbindungen gemäß einem oder mehreren der Ansprüche 1 bis 3 in Kombination mit mindestens einem weiteren anorektischen Wirkstoff zur Anwendung als Medikament zur Prophylaxe oder Behandlung der Obesitas.

9. Verbindungen gemäß einem oder mehreren der Ansprüche 1 bis 3 in Kombination mit mindestens einem weiteren anorektischen Wirkstoff zur Anwendung als Medikament zur Prophylaxe oder Behandlung der des Typ II Diabetes.

10. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen gemäß einem oder mehreren der Ansprüche 1 bis 3 zur Herstellung eines Medikaments zur Prophylaxe oder Behandlung der Obesitas.

12. Verwendung der Verbindungen gemäß einem oder mehreren der Ansprüche 1 bis 3 zur Herstellung eines Medikaments zur Prophylaxe oder Behandlung des Typ II Diabetes.

## Claims

1. A compound of the formula I 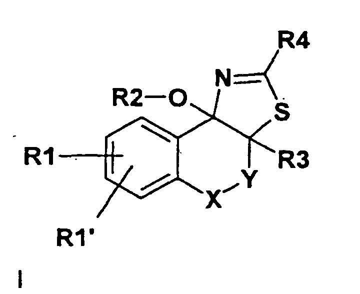 in which
Y is a direct linkage, -CH₂-, -CH₂-CH₂-;
X is CH₂, CH(CH₃), CH(C₂H₅), CH(C₃H₇), CH(C₆H₅);
R1, R1' are, independently of one another, H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl radicals can be replaced by fluorine, or one hydrogen can be replaced by OH, OC(O)CH₃, OC(O)H, O-CH₂-Ph, NH₂, NH-CO-CH₃ or N(COOCH₂Ph)₂;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n can be 0-6 and the phenyl radical can be substituted up to twice by F, Cl, Br, OH, CF₃, NO₂, CN, OCF₃, O-(C₁-C₆)-alkyl, (C₁-C₆)-alkyl or NH₂; NH₂, NH-(C₁-C₆)-alkyl, N((C₁-C₆)-alkyl)₂, NH(C₁-C₇)-acyl, phenyl, biphenylyl, O-(CH₂)ₙ-phenyl, where n can be 0-6, 1- or 2-naphthyl, 2-, 3- or 4-pyridyl, 2- or 3-furanyl or 2- or 3-thienyl, it being possible for the phenyl, biphenylyl, naphthyl, pyridyl, furanyl or thienyl rings each to be substituted up to 3 times by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl or CONH₂;
1,2,3-triazol-5-yl, it being possible for the triazol ring to be substituted in position 1, 2 or 3 by methyl or benzyl; tetrazol-5-yl, it being possible for the tetrazol ring to be substituted in position 1 or 2 by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0-5 and in which phenyl, thienyl, pyridyl, furyl can each be substituted up to twice by Cl, F, CN, CF₃, (C₁-C₃)-alkyl, OH or O-(C₁-C₆)-alkyl;
R3 is H, (C₁-C₆)-alkyl, F, CN, N₃, O-(C₁-C₆)-alkyl, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl, furyl can each be substituted up to twice by Cl, F, CN, CF₃, (C₁-C₃)-alkyl, OH or O-(C₁-C₆)-alkyl, or (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is NR6R7;
R6 and R7 are, independently of one another, H, (C₁-C₆)-alkyl, (C₃-C₆)-cycloalkyl, phenyl, it being possible for the phenyl ring to be substituted up to twice by Cl, F, CN, CF₃, (C₁-C₃)-alkyl, OH or O-(C₁-C₆)-alkyl;
CO-(C₁-C₆)-alkyl, CHO, CO-phenyl, -NH₂, -N=C(CH₃)₂, -(pyrrolidin-1-yl), -(piperidin-1-yl), -(morpholin-4-yl), -(piperazin-1-yl), -(4-methylpiperazin-1-yl),
or
NR6R7 is a ring such as pyrrolidine, piperidine, morpholine, piperazine, 4-methylpiperazin-1-yl, 4-benzylpiperazin-1-yl, phthalimidyl;
and its physiologically tolerated salts.

2. A compound of the formula I as claimed in claim 1, wherein the meanings are as follows
Y a direct linkage;
X CH₂;
R1, R1' independently of one another, H, F, Cl, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl radicals can be replaced by fluorine, or one hydrogen can be replaced by OH, OC(O)CH₃, OC(O)H, O-CH₂-Ph, NH₂, NH-CO-CH₃ or N(COOCH₂Ph)₂;
SO₂-NH₂, SO₂NH(C₁-C₆)-alkyl, SO₂N[(C₁-C₆)-alkyl]₂, SO₂-(C₁-C₆)-alkyl, SO₂-(CH₂)ₙ-phenyl, where n can be 0-6 and the phenyl radical can be substituted by F, Cl, Br, OH, CF₃, NO₂, CN, OCF₃, O-(C₁-C₆)-alkyl, (C₁-C₆)-alkyl or NH₂; NH₂, NH-(C₁-C₆)-alkyl, N((C₁-C₆)-alkyl)₂, NH(C₁-C₇)-acyl, phenyl, biphenylyl, O-(CH₂)ₙ-phenyl, where n can be 0-6, 1- or 2-naphthyl, 2-, 3- or 4-pyridyl, 2- or 3-furanyl or 2- or 3-thienyl, it being possible for the phenyl, biphenylyl, naphthyl, pyridyl, furanyl or thienyl rings each to be substituted once or twice by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl or CONH₂;
1,2,3-triazol-5-yl, it being possible for the triazole ring to be substituted in position 1, 2 or 3 by methyl or benzyl; tetrazol-5-yl, it being possible for the tetrazole ring to be substituted in position 1 or 2 by methyl or benzyl;
R2 is H, (C₁-C₆)-alkyl, (C₃-C₆)-cycloalkyl, (CH₂)ₙ-phenyl, (CH₂)ₙ-thienyl, (CH₂)ₙ-pyridyl, (CH₂)ₙ-furyl, C(O)-(C₁-C₆)-alkyl, C(O)-(C₃-C₆)-cycloalkyl, C(O)-(CH₂)ₙ-phenyl, C(O)-(CH₂)ₙ-thienyl, C(O)-(CH₂)ₙ-pyridyl, C(O)-(CH₂)ₙ-furyl, where n can be 0-5 and in which phenyl, thienyl, pyridyl, furyl can each be substituted up to twice by Cl, F, CN, CF₃, (C₁-C₃)-alkyl, OH or O-(C₁-C₆)-alkyl;
R3 is H, (C₁-C₆)-alkyl, F, (CH₂)ₙ-phenyl, (CH₂)ₙ-thienyl, (CH₂)ₙ-pyridyl, (CH₂)ₙ-furyl, where n can be 0-5 and in which phenyl, thienyl, pyridyl, furyl can each be substituted up to twice by Cl, F, CN, CF₃, (C₁-C₃)-alkyl, OH or O-(C₁-C₆)-alkyl, or (C₂-C₆)-alkynyl, (C₂-C₆)-alkenyl, C(O)OCH₃, C(O)OCH₂CH₃, C(O)OH, C(O)NH₂, C(O)NHCH₃, C(O)N(CH₃)₂, OC(O)CH₃;
R4 is NR6R7;
R6 and R7 are, independently of one another, H, (C₁-C₆)-alkyl, (C₃-C₆)-cycloalkyl, phenyl, it being possible for the phenyl ring to be substituted up to twice by Cl, F, CN, CF₃, (C₁-C₃)-alkyl, OH or O-(C₁-C₆)-alkyl;
CO-(C₁-C₆)-alkyl, CHO, CO-phenyl, -NH₂, -N=C(CH₃)₂, -(pyrrolidin-1-yl), -(piperidin-1-yl), -(morpholin-4-yl),-(piperazin-1-yl), -(4-methylpiperazin-1-yl),
or
NR6R7 is a ring such as pyrrolidine, piperidine, morpholine, piperazine, 4-methylpiperazin-1-yl, 4-benzylpiperazin-1-yl, phthalimidyl;
and its physiologically tolerated salts.

3. A compound of the formula I as claimed in claim 1 or 2, wherein the meanings are as follows
Y a direct linkage;
X CH₂;
R1, R1' are, independently of one another, H, F, Cl, CF₃, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more than one or all hydrogen(s) in the alkyl radicals can be replaced by fluorine, or one hydrogen can be replaced by OH, OC(O)CH₃, OC(O)H, O-CH₂-Ph, NH₂, NH-CO-CH₃ or N(COOCH₂Ph)₂;
SO₂-(C₁-C₆)-alkyl, SO₂-(CH₂)ₙ-phenyl, where n can be 0-3 and the phenyl radical can be substituted by F, Cl, OH, CF₃, CN, OCF₃, O-(C₁-C₆)-alkyl, (C₁-C₆)-alkyl or NH₂;
NH₂, NH-(C₁-C₆)-alkyl, N((C₁-C₆)-alkyl)₂, NH(C₁-C₇)-acyl, phenyl, biphenylyl, O-(CH₂)ₙ-phenyl, where n can be 0-6, 1- or 2-naphthyl, 2-, 3- or 4-pyridyl, 2- or 3-furanyl or 2- or 3-thienyl, it being possible for the phenyl, biphenylyl, naphthyl, pyridyl, furanyl or thienyl rings each to be substituted once or twice by F, Cl, OH, CF₃, CN, OCF₃, O-(C₁-C₄)-alkyl, (C₁-C₄)-alkyl, NH₂, NH(C₁-C₄)-alkyl, N((C₁-C₄)-alkyl)₂, SO₂-CH₃, COOH, COO-(C₁-C₄)-alkyl, or CONH₂;
1,2,3-triazol-5-yl, it being possible for the triazole ring to be substituted in position 1, 2 or 3 by methyl or benzyl; tetrazol-5-yl, it being possible for the tetrazole ring to be substituted in position 1 or 2 by methyl or benzyl;
R2 is H, (C₁-C₆)-alkyl, (CH₂)ₙ-phenyl, (CH₂)ₙ-thienyl, (CH₂)ₙ-pyridyl, (CH₂)ₙ-furyl, in which phenyl, thienyl, pyridyl, furyl can each be substituted up to twice by Cl, F, CN, CF₃, (C₁-C₃)-alkyl, OH or O-(C₁-C₆)-alkyl;
R3 is H, F; (C₁-C₄)-alkyl;
R4 is NR6R7;
R6 and R7 are, independently of one another, H, (C₁-C₆)-alkyl, (C₃-C₆)-cycloalkyl, phenyl, it being possible for the phenyl ring to be substituted up to twice by Cl, F, CN, CF₃, (C₁-C₃)-alkyl, OH or O-(C₁-C₆)-alkyl;
CO-(C₁-C₆)-alkyl, CHO, CO-phenyl, -NH₂, -N=C(CH₃)₂, -(pyrrolidin-1-yl), -(piperidin-1-yl), -(morpholin-4-yl), -(piperazin-1-yl), -(4-methylpiperazin-1-yl),
or
NR6R7 is a ring such as pyrrolidine, piperidine, morpholine, piperazine, 4-methylpiperazin-1-yl, 4-benzylpiperazin-1-yl, phthalimidyl; and its physiologically tolerated salts.

4. A pharmaceutical comprising one or more compounds as claimed in one or more of claims 1 to 3.

5. A pharmaceutical comprising one or more compounds as claimed in one or more of claims 1 to 3 and one or more anorectic active ingredients.

6. A compound as claimed in one or more of claims 1 to 3 for use as medicine for the prophylaxis or treatment of obesity.

7. A compound as claimed in one or more of claims 1 to 3 for use as medicine for the prophylaxis or treatment of type II diabetes.

8. A compound as claimed in one or more of claims 1 to 3 in combination with at least one other anorectic active ingredient for use as medicine for the prophylaxis or treatment of obesity.

9. A compound as claimed in one or more of claims 1 to 3 in combination with at least one other anorectic active ingredient for use as medicine for the prophylaxis or treatment of type II diabetes.

10. A process for preparing a pharmaceutical comprising one or more compounds as claimed in one or more of claims 1 to 3, which comprises mixing the active ingredient with a pharmaceutically suitable carrier and converting this mixture into a form suitable for administration.

11. The use of the compounds as claimed in one or more of claims 1 to 3 for producing a medicine for the prophylaxis or treatment of obesity.

12. The use of the compounds as claimed in one or more of claims 1 to 3 for producing a medicine for the prophylaxis or treatment of type II diabetes.

## Revendications

1. Composés de formule I, 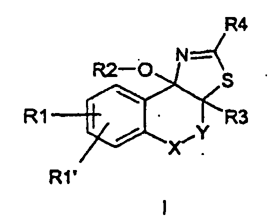 dans laquelle
Y représente une liaison directe, -CH₂-, -CH₂-CH₂- ;
X représente CH₂, CH(CH₃), CH(C₂H₅), CH(C₃H₇), CH(C₆H₅) ;
R1, R1' représentent, indépendamment l'un de l'autre H, F, Cl, Br, I, CF₃, NO₂, CN, COOH, COO(C₁-C₆)alkyle, CONH₂, CONH(C₁-C₆)alkyle, CON[(C₁-C₆)alkyle)]₂, un alkyle en C₁-C₆, un alcényle en C₂-C₆, un alcynyle en C₂-C₆, un O-(C₁-C₆)-alkyle, où dans les radicaux alkyle, un, plusieurs ou la totalité des hydrogènes peuvent être remplacés par un fluor, ou un hydrogène peut être remplacé par OH, OC(O)CH₃, OC(O)H, O-CH₂-Ph, NH₂, NH-CO-CH₃ ou N(COOCH₂Ph)₂ ;
SO₂-NH₂, un SO₂NH(C₁-C₆)alkyle, un SO₂N[(C₁-C₆)-alkyle]₂, un S-(C₁-C₆)alkyle, un S-(CH₂)ₙ-phényle, un SO-(C₁-C₆)alkyle, un SO-(CH₂)ₙ-phényle, un SO₂(C₁-C₆)alkyle, un SO₂-(CH₂)ₙ-phényle, où n peut valoir de 0 à 6 et le radical phényle peut être jusqu'à bi-substitué par F, Cl, Br, OH, CF₃, NO₂, CN, OCF₃, un O-(C₁-C₆)alkyle, un alkyle en C₁-C₆ ou NH₂ ; NH₂, un NH-(C₁-C₆)alkyle, un N ((C₁-C₆)alkyle)₂, un NH(C₁-C₇)alcyle, un phényle, un biphénylyle, un O-(CH₂)ₙ-phényle, où n peut valoir de 0 à 6, un 1- ou 2-naphtyle, un 2-, 3- ou 4-pyridyle, un 2- ou 3-furanyle ou un 2-ou 3-thiényle, où les noyaux phényle, biphénylyle, naphtyle, pyridyle, furanyle ou thiényle peuvent chacun être mono- à tri-substitués par F, Cl, Br, I, OH, CF₃, NO₂, CN, OCF₃, un O-(C₁-C₆)alkyle, un alkyle en C₁-C₆, NH₂, un NH(C₁-C₆)alkyle, un N((C₁-C₆)alkyle)₂, SO₂CH₃, COOH, COO(C₁-C₆)alkyle, ou CONH₂ ;
un 1,2,3-triazol-5-yle où le noyau triazole peut être substitué en position 1, 2 ou 3 par un méthyle ou un benzyle ;
un tétrazol-5-yle, où le noyau tétrazole peut être substitué en position 1 ou 2 par un méthyle ou un benzyle ;
R2 représente H, un alkyle en C₁-C₆, un cycloalkyle en C₃-C₆, un (CH₂)ₙ-phényle, un (CH₂)ₙ-thiényle, un (CH₂)ₙ-pyridyle, un (CH₂)ₙ-furyle, un C(O)-(C₁-C₆)alkyle, un C(O)-(C₃-C₆)cycloalkyle, un C(O)-(CH₂)ₙ-phényle, un C(O)-(CH₂)ₙ-thiényle, un C(O)-(CH₂)ₙ-pyridyle, un C(O)-(CH₂)ₙ-furyle, où n peut valoir de 0 à 5 et où le phényle, le thiényle, le pyridyle ou le furyle peut être jusqu'à bi-substitué par Cl, F, CN, CF₃, un alkyle en C₁-C₃, OH ou un O-(C₁-C₆)alkyle ;
R3 représente H, un alkyle en C₁-C₆, F, CN, N₃, un O-(C₁-C₆)alkyle, un (CH₂)ₙ-phényle, un (CH₂)ₙ-thiényle, un (CH₂)ₙ-pyridyle, un (CH₂)ₙ-furyle, où n peut valoir de 0 à 5 et où le phényle, le thiényle, le pyridyle ou le furyle peut être jusqu'à bi-substitué par Cl, F, CN, CF₃, un alkyle en C₁-C₃, OH ou un O-(C₁-C₆)alkyle, un alcynyle en C₂-C₆, un alcényle en C₂-C₆, C(O)OCH₃, C(O)OCH₂CH₃, C(O)OH, C(O)NH₂, C(O)NHCH₃, C(O)N(CH₃)₂, OC(O)CH₃ ;
R4 représente NR6R7 ;
R6 et R7 représentent indépendamment l'un de l'autre H, un alkyle en C₁-C₆, un cycloalkyle en C₃-C₆, un phényle, où le noyau phényle peut être jusqu'à bi-substitué par Cl, F, CN, CF₃, un alkyle en C₁-C₃, OH ou un O-(C₁-C₆)alkyle ; un CO-(C₁-C₆)alkyle, CHO, un CO-phényle, -NH₂, -N=C(CH₃)₂, un -(pyrrolidin-1-yle), un -(pipéridin-1-yle), un -morpholin-4-yle), -(pipérazin-1-yle), un -(4-méthylpipérazin-1-yle),
ou
NR6R7 représente un noyau tel qu'une pyrrolidine, une pipéridine, une morpholine, une pipérazine, un N-4-méthyl-pipérazin-1-yle, un N-4-benzyl-pipérazin-1-yle, un phtalimidyle ;
ainsi que leurs sels physiologiquement acceptables.

2. Composés de formule I, selon la revendication 1, **caractérisés en ce que**
Y représente une liaison directe ;
X représente CH₂ ;
R1, R1' représentent, indépendamment l'un de l'autre H, F, Cl, CF₃, NO₂, CN, COOH, COO(C₁-C₆)alkyle, CONH₂, CONH(C₁-C₆)alkyle, CON[(C₁-C₆)alkyle]₂, un alkyle en C₁-C₆, un alcényle en C₂-C₆, un alcynyle en C₂-C₆, un O-(C₁-C₆)-alkyle, où dans les radicaux alkyle, un, plusieurs ou la totalité des hydrogènes peuvent être remplacés par un fluor, ou un hydrogène peut être remplacé par OH, OC(O)CH₃, OC(O)H, O-CH₂-Ph, NH₂, NH-CO-CH₃ ou N(COOCH₂Ph)₂ ;
SO₂-NH₂, un SO₂NH(C₁-C₆)alkyle, un SO₂N[(C₁-C₆)-alkyle]₂, un SO₂(C₁-C₆)-alkyle, un SO₂(CH₂)ₙ-phényle, où n peut valoir de 0 à 6 et le radical phényle peut être jusqu'à bi-substitué par F, Cl, Br, OH, CF₃, NO₂, CN, OCF₃, un O-(C₁-C₆)alkyle, un alkyle en C₁-C₆ ou NH₂ ; NH₂, un NH-(C₁-C₆)alkyle, un N ((C₁-C₆)alkyle)₂, un NH(C₁-C₇)alcyle, un phényle, un biphénylyle, un O-(CH₂)ₙ-phényle, où n peut valoir de 0 à 6, un 1- ou 2-naphtyle, un 2-, 3- ou 4-pyridyle, un 2- ou 3-furanyle ou un 2-ou 3-thiényle, où les noyaux phényle, biphénylyle, naphtyle, pyridyle, furanyle ou thiényle peuvent chacun être mono- à bi-substitués par F, Cl, Br, I, OH, CF₃, NO₂, CN, OCF₃, un O-(C₁-C₆)alkyle, un alkyle en C₁-C₆, NH₂, un NH (C₁-C₆)alkyle, un N((C₁-C₆)alkyle)₂, SO₂CH₃, COOH, COO(C₁-C₆)alkyle, ou CONH₂ ;
un 1,2,3-triazol-5-yle où le noyau triazole peut être substitué en position 1, 2 ou 3 par un méthyle ou un benzyle ;
un tétrazol-5-yle, où le noyau tétrazole peut être substitué en position 1 ou 2 par un méthyle ou un benzyle ;
R2 représente H, un alkyle en C₁-C₆, un cycloalkyle en C₃-C₆, un (CH₂)ₙ-phényle, un (CH₂)ₙ-thiényle, un (CH₂)ₙ-pyridyle, un (CH₂)ₙ-furyle, un C(O)-(C₁-C₆)alkyle, un C(O)-(C₃-C₆)cycloalkyle, un C(O)-(CH₂)ₙ-phényle, un C(O)-(CH₂)ₙ-thiényle, un C(O)-(CH₂)ₙ-pyridyle, un C(O)-(CH₂)ₙ-furyle, où n peut valoir de 0 à 5 et où le phényle, le thiényle, le pyridyle ou le furyle peut être jusqu'à bi-substitué par Cl, F, CN, CF₃, un alkyle en C₁-C₃, OH ou un O-(C₁-C₆)alkyle ;
R3 représente H, un alkyle en C₁-C₆, F, un (CH₂)ₙ-phényle, un (CH₂)ₙ-thiényle, un (CH₂)ₙ-pyridyle, un (CH₂)ₙ-furyle, où n peut valoir de 0 à 5 et où le phényle, le thiényle, le pyridyle ou le furyle peut être jusqu'à bi-substitué par Cl, F, CN, CF₃, un alkyle en C₁-C₃, OH ou un O-(C₁-C₆)alkyle, un alcynyle en C₂-C₆, un alcényle en C₂-C₆, C(O)OCH₃, C(O)OCH₂CH₃, C(O)OH, C(O)NH₂, C(O)NHCH₃, C(O)N(CH₃)₂, OC(O)CH₃ ;
R4 représente NR6R7 ;
R6 et R7 représentent indépendamment l'un de l'autre H, un alkyle en C₁-C₆, un cycloalkyle en C₃-C₆, un phényle, où le noyau phényle peut être jusqu'à bi-substitué par Cl, F, CN, CF₃, un alkyle en C₁-C₃, OH ou un O-(C₁-C₆)alkyle ; un CO-(C₁-C₆)alkyle, CHO, un CO-phényle, -NH₂, -N=C(CH₃)₂, un -(pyrrolidin-1-yle), un -(pipéridin-1-yle), un -morpholin-4-yle), -(pipérazin-1-yle), un -(4-méthylpipérazin-1-yle),
ou
NR6R7 représente un noyau tel qu'une pyrrolidine, une pipéridine, une morpholine, une pipérazine, un N-4-méthyl-pipérazin-1-yle, un N-4-benzyl-pipérazin-1-yle, un phtalimidyle ;
ainsi que leurs sels physiologiquement acceptables.

3. Composés de formule générale I, selon la revendication 1 ou 2, **caractérisés en ce que**
Y représente une liaison directe ;
X représente CH₂ ;
R1, R1' représentent, indépendamment l'un de l'autre H, F, Cl, CF₃, CN, COOH, un COO(C₁-C₆)alkyle, CONH₂, un CONH (C₁-C₆)alkyle, un CON[(C₁-C₆)alkyle]₂, un alkyle en C₁-C₆, un alcényle en C₂-C₆, un alcynyle en C₂-C₆, un O-(C₁-C₆)-alkyle, où dans les radicaux alkyle, un, plusieurs ou la totalité des hydrogènes peuvent être remplacés par un fluor, ou un hydrogène peut être remplacé par OH, OC(O)CH₃, OC(O)H, O-CH₂-Ph, NH₂, NH-CO-CH₃ ou N(COOCH₂Ph)₂ ;
un SO₂(C₁-C₆)-alkyle, un SO₂(CH₂)ₙ-phényle, où n peut valoir de 0 à 3 et le radical phényle peut être remplacé par F, Cl, OH, CF₃, CN, OCF₃, un O-(C₁-C₆)alkyle, un alkyle en C₁-C₆ ou NH₂ ; NH₂, un NH-(C₁-C₆)alkyle, un N((C₁-C₆)alkyle)₂, un NH((C₁-C₇)alcyle, un phényle, un biphénylyle, un O-(CH₂)ₙ-phényle, où n peut valoir de 0 à 6, un 1- ou 2-naphtyle, un 2-, 3- ou 4-pyridyle, un 2- ou 3-furanyle ou un 2-ou 3-thiényle, où les noyaux phényle, biphénylyle, naphtyle, pyridyle, furanyle ou thiényle peuvent chacun être mono- à bi-substitués par F, Cl, OH, CF₃, CN, OCF₃, un O-(C₁-C₄)alkyle, un alkyle en C₁-C₄, NH₂, un NH(C₁-C₄)alkyle, un N((C₁-C₄)alkyle)₂, SO₂CH₃, COOH, un COO(C₁-C₄)-alkyle, ou CONH₂ ;
un 1,2,3-triazol-5-yle où le noyau triazole peut être substitué en position 1, 2 ou 3 par un méthyle ou un benzyle ;
un tétrazol-5-yle, où le noyau tétrazole peut être substitué en position 1 ou 2 par un méthyle ou un benzyle ;
R2 représente H, un alkyle en C₁-C₆, un (CH₂)ₙ-phényle, un (CH₂)ₙ-thiényle, un (CH₂)ₙ-pyridyle, un (CH₂)ₙ-furyle, où le phényle, le thiényle, le pyridyle ou le furyle peuvent chacun être jusqu'à bi-substitués par Cl, F, CN, CF₃, un alkyle en C₁-C₃, OH ou un O-(C₁-C₆)alkyle ;
R3 représente H, F, un alkyle en C₁-C₄ ;
R4 représente NR6R7 ;
R6 et R7 représentent indépendamment l'un de l'autre H, un alkyle en C₁-C₆, un cycloalkyle en C₃-C₆, un phényle, où le noyau phényle peut être jusqu'à bi-substitué par Cl, F, CN, CF₃, un alkyle en C₁-C₃, OH ou un O-(C₁-C₆)alkyle ; un CO-(C₁-C₆)alkyle, CHO, un CO-phényle, -NH₂, -N=-C(CH₃)₂, un - (pyrrolidin-1-yle), un -(pipéridin-1-yle), un -morpholin-4-yle), -(pipérazin-1-yle), un -(4-méthylpipérazin-1-yle),
ou
NR6R7 représente un noyau tel qu'une pyrrolidine, une pipéridine, une morpholine, une pipérazine, un N-4-méthyl-pipérazin-1-yle, un N-4-benzyl-pipérazin-1-yle, un phtalimidyle ;
ainsi que leurs sels physiologiquement acceptables.

4. Médicament comprenant un ou plusieurs des composés selon l'une ou plusieurs des revendications 1 à 3.

5. Médicament comprenant un ou plusieurs des composés selon l'une ou plusieurs des revendications 1 à 3 et un ou plusieurs ingrédients actifs anorexiants.

6. Composés selon l'une ou plusieurs des revendications 1 à 3 destinés à être utilisés en tant que médicament pour la prophylaxie ou le traitement de l'obésité.

7. Composés selon l'une ou plusieurs des revendications 1 à 3 destinés à être utilisés en tant que médicament pour la prophylaxie ou le traitement du diabète de type II.

8. Composés selon l'une ou plusieurs des revendications 1 à 3 en combinaison avec au moins un autre ingrédient actif anorexiant destinés à être utilisés en tant que médicament pour la prophylaxie ou le traitement de l'obésité.

9. Composés selon l'une ou plusieurs des revendications 1 à 3 en combinaison avec au moins un autre ingrédient actif anorexiant destinés à être utilisés en tant que médicament pour la prophylaxie ou le traitement du diabète de type II.

10. Procédé de production d'un médicament comprenant un ou plusieurs des composés selon l'une ou plusieurs des revendications 1 à 3, **caractérisé en ce que** l'ingrédient actif est mélangé avec un support pharmaceutiquement approprié et ce mélange est mis sous une forme appropriée pour l'administration.

11. Utilisation des composés selon l'une ou plusieurs des revendications 1 à 3 pour la production d'un médicament destiné à la prophylaxie ou au traitement de l'obésité.

12. Utilisation des composés selon l'une ou plusieurs des revendications 1 à 3 pour la production d'un médicament destiné à la prophylaxie ou au traitement du diabète de type II.
